Europäisches Patentamt

European Patent Office

Office européen des brevets

(19)

(11) Veröffentlichungsnummer: **0 220 665**

**A2**

(12) **EUROPÄISCHE PATENTANMELDUNG**

(21) Anmeldenummer: 86114590.2

(22) Anmeldetag: 21.10.86

(51) Int. Cl.4: **C07K 5/00** , A61K 37/02 , C07D 233/64

(30) Priorität: 31.10.85 DE 3538749

(43) Veröffentlichungstag der Anmeldung: 06.05.87 Patentblatt 87/19

(84) Benannte Vertragsstaaten: AT BE CH DE FR GB IT LI NL SE

(71) Anmelder: **Merck Patent Gesellschaft mit beschränkter Haftung Frankfurter Strasse 250 D-6100 Darmstadt(DE)**

(72) Erfinder: **Raddatz, Peter, Dr. Grafenstrasse 37 D-6100 Darmstadt(DE)**
Erfinder: **Hölzemann, Günter, Dr. Weedring 5 D-6104 Seeheim 1(DE)**
Erfinder: **Jonczyk, Alfred, Dr. Eschelkopfweg 5 D-6100 Darmstadt(DE)**
Erfinder: **Schmitges, Claus J., Dr. Karolinger Strasse 5 D-6114 Gross-Umstadt(DE)**
Erfinder: **Minck, Klaus Otto, Dr. Büchestrasse 8 D-6105 Ober-Ramstadt(DE)**

(54) **Peptide.**

(57) Neue Peptide der Formel I

X-Z-NR²-CHR³-CHOH-(CHR⁴)ₙ-CO-E I

worin

X, Z, R², R³, R⁴, E und n die in Patentanspruch 1 angegebene Bedeutung haben,

sowie ihre Salze hemmen die Aktivität des menschlichen Plasmarenins.

EP 0 220 665 A2

# Peptide

Die Erfindung betrifft neue Peptide der Formel I

$$X-Z-NR^2-CHR^3-CHOH-(CHR^4)_n-CO-E \quad I$$

worin

X H, $R^1-O-C_mH_{2m}-CO-$, $R^1-C_mH_{2m}-O-CO-$, $R^1-C_mH_{2m}-CO-$, $R^1-SO_2-$, $(R^1-C_mH_{2m})-L(R^1-C_pH_{2p})-C_r H_{2r}-CO-$, $H-(NHCH_2CH_2)_m-NH-CH_2CO-$ oder 9-Fluorenyl-$C_mH_{2m}-O-CO-$,

Z 1 bis 4 peptidartig miteinander verbundene Aminosäurereste ausgewählt aus der Gruppe bestehend aus Abu, Ada, Ala, Arg, Asn, Bia, Cal, Dab, Gln, Gly, His, N(im)-Alkyl-His, Ile, Leu, tert.-Leu, Lys, Met, αNal, βNal, Nbg, Nle, Orn, Phe, Pro, Ser, Thr, Tic, Trp, Tyr und Val,

E OH, OA, $NH_2$, NHA oder $NA_2$,

$R^1$ H, A, Ar, Ar-alkyl, unsubstituiertes oder ein-oder mehrfach durch Alkyl, Alkoxy und/oder Hal substituiertes Cycloalkyl mit 3-7 C-Atomen, Cycloalkylalkyl mit 4-11 C-Atomen, Bicycloalkyl oder Tricycloalkyl mit jeweils 7-14 C-Atomen, Bicycloalkylalkyl oder Tricycloalkylalkyl mit jeweils 8-18 C-Atomen, wobei die Gruppe$(R^1-C_mC_{2m})-L(R^1-C_pC_{2p})$ auch Pyrrolidino, Piperidino, Morpholino oder Thiomorpholino bedeuten kann,

$R^2$ und $R^4$ jeweils H oder A,

$R^3$ Cycloalkylalkyl, Bicycloalkylalkyl oder Tricycloalkylalkyl mit jeweils bis zu 18 C-Atomen,

L CH oder N,

m, p und r jeweils 0, 1, 2, 3, 4 oder 5,

n 1 oder 2,

Ar unsubstituiertes oder ein-oder mehrfach durch A, AO, Hal, $CF_3$, OH und/oder $NH_2$ substituiertes Phenyl oder unsubstituiertes Naphthyl,

Hal F, Cl, Br oder J und

A Alkyl mit 1-8 C-Atomen bedeuten,

worin ferner an Stelle einer oder mehrerer -NH-CO-Gruppen auch eine oder mehrere -N(Alkyl)-CO-Gruppen stehen können,

sowie deren Salze.

Ähnliche Verbindungen sind aus der EP-A-77028 bekannt.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze sehr wertvolle Eigenschaften besitzen. Vor allem hemmen sie die Aktivität des menschlichen Plasma renins. Diese Wirkung kann z. B. nach der Methode von F. Fyhrquist et. al., Clin.Chem. 22, 250-256 (1976), nachgewiesen werden. Bemerkenswert ist, daß diese Verbindungen sehr spezifische Hemmer des Renins sind; für die Hemmung anderer Aspartylproteinasen (z. B. Pepsin und Kathepsin D) sind in der Regel wesentlich höhere Konzentrationen dieser Verbindungen notwendig.

Die Verbindungen können als Arzneimittelwirkstoffe in der Human-und Veterinärmedizin eingesetzt werden, insbesondere zur Prophylaxe und zur Behandlung von Herz-, Kreislauf-und Gefäßkrankheiten, vor allem von Hypertonie, Herzinsuffizienz und Hyperaldosteronismus. Außerdem können die Verbindungen zu diagnostischen Zwecken verwendet werden, um bei Patienten mit Hypertonie oder Hyperaldosteronismus den möglichen Beitrag der Reninaktivität zur Aufrechterhaltung des pathologischen Zustands zu bestimmen.

Die vor-und nachstehend aufgeführten Abkürzungen von Aminosäureresten stehen für die Reste -NH-CHR-CO-(worin R die für jede Aminosäure bekannte spezifische Bedeutung hat) folgender Aminosäuren:

Abu 2-Aminobuttersäure

Ada Adamantylalanin

Ala Alanin

Arg Arginin

Asn Asparagin

Bia Benzimidazolylalanin

Cal Cyclohexylalanin

Dab 2,4-Diaminobuttersäure

Gln Glutamin

Gly Glycin

His Histidin

N(im)-Alkyl-His in 1-oder 3-Stellung des Imidazolrings durch A substituiertes Histidin

Ile Isoleucin

Leu Leucin

tert.-Leu tert.-Leucin

Lys Lysin

Met Methionin

αNal α-Naphthylalanin

βNal β-Naphthylalanin

Nbg (2-Norbornyl)-glycin

Nle Norleucin

N-Me-His N-Methyl-histidin

N-Me-Phe N-Methyl-phenylalanin

Orn Ornithin

Phe Phenylalanin

Pro Prolin

Ser Serin

Thr Threonin

Tic Tetrahydroisochinolin-1-carbonsäure

Trp Tryptophan

Tyr Tyrosin

Val Valin.

Ferner bedeutet nachstehend:

BOC tert.-Butoxycarbonyl

imi-BOM Benzyloxymethyl in 1-Stellung des Imidazolrings

CBZ Benzyloxycarbonyl

DNP 2,4-Dinitrophenyl

imi-DNP 2,4-Dinitrophenyl in 1-Stellung des Imidazolrings

FMOC 9-Fluorenylmethoxycarbonyl

OMe Methylester

OEt Ethylester

POA Phenoxyacetyl

DCCI Dicyclohexylcarbodiimid

HOBt 1-Hydroxybenzotriazol.

Sofern die vorstehend genannten Aminosäuren in mehreren enantiomeren Formen auftreten können, so sind vor-und nachstehend, z. B. als Bestandteil der Verbindungen der Formel I, alle diese Formen und auch ihre Gemische (z. B. die DL-Formen) eingeschlossen. Die L-Formen sind bevorzugt. Sofern nachstehend einzelne Verbindungen aufgeführt sind, so beziehen sich die Abkürzungen dieser Aminosäuren jeweils auf die L-Form, sofern nicht ausdrücklich etwas anderes angegeben ist.

Vor-und nachstehend haben die Reste bzw. Parameter X, Z, E, $R^1$ bis $R^4$, L, m, n, p, r, Ar, Hal, A, $G^1$, $G^2$, $Z^1$, $Z^2$ und W die bei den Formeln I, II oder III angegebenen Bedeutungen, falls nicht ausdrücklich etwas anderes angegeben ist.

In den vorstehenden Formeln hat A 1 -8, vorzugsweise 1,2,3 oder 4 C-Atome. A bedeutet vorzugsweise Methyl, weiterhin Ethyl, Propyl, Isopropyl, Butyl, Isobutyl, sek.-Butyl oder tert.-Butyl, ferner auch Pentyl, 1-, 2-oder 3-Methylbutyl, 1,1-, 1,2-oder 2,2-Dimethylpropyl, 1-Ethylpropyl, Hexyl, 1-, 2-, 3-oder 4-Methylpentyl, 1,1-, 1,2-, 1,3-, 2,2-, 2,3-oder 3,3-Dimethylbutyl, 1-oder 2-Ethylbutyl, 1-Ethyl-1-methylpropyl, 1-Ethyl-2-methylpropyl, 1,1,2-oder 1,2,2-Trimethylpropyl.

Cycloalkyl bedeutet vorzugsweise Cyclopropyl, Cyclobutyl, Cyclopentyl, Cyclohexyl oder Cycloheptyl, aber auch z. B. 1-, 2-oder 3-Methylcyclopentyl, 1-, 2-, 3-oder 4-Methylcyclohexyl.

Dementsprechend bedeutet Cycloalkyl-alkyl vorzugsweise Cyclopropylmethyl, 2-Cyclopropylethyl, Cyclobutylmethyl, 2-Cyclobutylethyl, Cyclopentylmethyl, 2-Cyclopentylethyl, Cyclohexylmethyl, 2-Cyclohexylethyl, aber auch z. B. 1-, 2-oder 3-Methylcyclopentylmethyl, 1-, 2-, 3-oder 4-Methylcyclohexylmethyl.

Bicycloalkyl bedeutet vorzugsweise 1-oder 2-Dekalyl, 2-Bicyclo[2,2,1]heptyl oder 6,6-Dimethyl-2-bicyclo [3,1,1]heptyl.

Tricycloalkyl bedeutet vorzugsweise 2-Adamantyl.

Ar bedeutet vorzugsweise Phenyl, ferner bevorzugt o-, m-oder p-Tolyl, o-, m-oder p-Ethylphenyl, o-, m-oder p-Methoxyphenyl, o-, m-oder p-Fluorphenyl, o-, m-, oder p-Chlorphenyl, o-, m-oder p-Bromphenyl, o-, m-oder p-Jodphenyl, o-, m-oder p-Trifluormethylphenyl, 2,3-, 2,4-, 2,5-, 2,6-, 3,4-oder 3,5-Dimethoxyphenyl, 3,4,5-Trimethoxyphenyl, o-, m-oder p-Aminophenyl, 1-oder 2-Naphthyl.

R¹ bedeutet vorzugsweise A, insbesondere Methyl, Ethyl, Propyl, Isopropyl, Butyl, Isobutyl oder tert.-Butyl, weiterhin vorzugsweise Cyclopropyl, Cyclopentyl, Cyclohexyl, Phenyl oder Benzyl.

R² und R⁴ bedeuten vorzugsweise H oder Methyl, ferner Ethyl, Propyl, Isopropyl, Butyl oder Isobutyl.

R³ bedeutet vorzugsweise Cyclohexylmethyl, ferner bevorzugt 2-Cyclohexylethyl, Bicyclo[2,2,1]-heptyl-2-methyl oder 6,6-Dimethylbicyclo[3,1,1]-heptyl-2-methyl.

m, p und r sind vorzugsweise 0, 1 oder 2; n ist vorzugsweise 1.

X bedeutet vorzugsweise H, POA, Alkoxycarbonyl wie BOC, CBZ, Alkanoyl wie Acetyl, Propionyl, Butyryl oder Isobutyryl, Cycloalkylcarbonyl wie Cyclopentylcarbonyl oder Cyclohexylcarbonyl, Aroyl wie Benzoyl, Arylalkanoyl wie Phenylacetyl, 2-oder 3-Phenylpropionyl, 4-Phenylbutyryl, 2-Benzyl-3-phenylpropionyl, 2-Benzyl-4-phenylbutyryl, 2-(2-Phenylethyl)-4-phenylbutyryl, 2-(2-Naphthylmethyl)-4-phenylbutyryl, 2-oder 3-o-, -m-oder -p-Fluorphenylpropionyl, 2-oder 3-o-, -m-oder -p-Chlorphenylpropionyl, Cycloalkylalkanoyl wie Cyclohexylacetyl, 2-oder 3-Cyclohexylpropionyl. Besonders bevorzugte Reste X sind H und BOC, ferner POA, 4-Phenylbutyryl, 2-Benzyl-3-phenylpropionyl, · 2-Benzyl-4-phenylbutyryl, 2-(2-Phenylethyl)-4-phenylbutyryl), 2-(2-Naphthylmethyl)-4-phenylbutyryl und CBZ.

Z bedeutet vorzugsweise 2, aber auch 1, weiterhin 3 oder 4 peptidartig miteinander verbundene Aminosäurereste, insbesondere eine der Gruppen His, Phe-His, Pro-Phe-His oder His-Pro-Phe-His, ferner bevorzugt die Gruppen Abu, Ada, Asn, Bia, Cal, Gln, N-(im)-Alkyl-His, Leu, αNal, βNal, Nle, Phe, Trp, Tyr Abu-His, Ada-His, Ala-His, Ala-Phe, Arg-His, Asn-His, Bia-His, Cal-His, Dab-His, Gly-His, His-His, Ile-His, Leu-His, tert.-Leu-His, Lys-His, Met-His, αNal-His, βNal-His, Nbg-His, Nle-His, (N-Me-His)-His, (N-Me-Phe)-His, Orn-His, Phe-Abu, Phe-Ada, Phe-Ala, Phe-Arg, Phe-Asn, Phe-Bia, Phe-Cal, Phe-Dab, Phe-Gln, Phe-Gly, Phe-(N-im-Alkyl-His), Phe-Ile, Phe-Leu, Phe-tert.-Leu, Phe-Lys, Phe-Met, Phe-α-Nal, Phe-βNal, Phe-Nbg, Phe-Nle, Phe-(N-Me-His), Phe-(N-Me-Phe), Phe-Orn, Phe-Phe, Phe-Pro, Phe-Ser, Phe-Thr, Phe-Tic, Phe-Trp, Phe-Tyr, Phe-Val, Pro-His, Ser-His, Thr-His, Tic-His, Trp-His, Tyr-His, Val-His, ferner Ada-Phe-His, Pro-Ala-His, Pro-Ala-Phe, Pro-Phe-Ala, Pro-Phe-Phe, His-Pro-Ala-His, His-Pro-Ala-Phe, His-Pro-Phe-Ala, His-Pro-Phe-Phe, weiterhin Pro-Abu-His, Pro-Ada-His, Pro-Arg-His, Pro-Asn-His, Pro-Bia-His, Pro-Dab-His, Pro-Gly-His, Pro-His-His, Pro-Ile-His, Pro-Leu-His, Pro-tert.-Leu-His, Pro-Lys-His, Pro-Met-His, Pro-Nbg-His, Pro-Nle-His, Pro-(N-Me-His)-His, Pro-(N-Me-Phe)-His, Pro-Orn-His, Pro-Phe-Abu, Pro-Phe Ada, Pro-Phe-Arg, Pro-Phe-Asn, Pro-Phe-Bia, Pro-Phe-Dab, Pro-Phe-Gln, Pro-Phe-Gly, Pro-Phe-(N-im-Alkyl-His), Pro-Phe-Ile, Pro-Phe-Leu, Pro-Phe-tert.-Leu, Pro-Phe-Lys, Pro-Phe-Met, Pro-Phe-Nbg, Pro-Phe-Nle, Pro-Phe-(N-Me-His), Pro-Phe-(N-Me-Phe), Pro-Phe-Orn, Pro-Phe-Pro, Pro-Phe-Ser, Pro-Phe-Thr, Pro-Phe-Tic, Pro-Phe-Trp, Pro-Phe-Tyr, Pro-Phe-Val, Pro-Pro-His, Pro-Ser-His, Pro-Thr-His, Pro-Tic-His, Pro-Trip-His, Pro-Tyr-His, Pro-Val-His, His-Pro-Abu-His, His-Pro-Ada-His, His-Pro-Arg-His, His-Pro-Asn-His, His-Pro-Bia-His, His-Pro-Dab-His, His-Pro-Gly-His, His-Pro-His-His, His-Pro-Ile-His, His-Pro-Leu-His, His-Pro-tert.-Leu-His, His-Pro-Lys-His, His-Pro-Met-His, His-Pro-Nbg-His, His-Pro-Nle-His, His-Pro-(N-Me-His)-His, His-Pro-(N-Me-Phe)-His, His-Pro-Orn-His, His-Pro-Phe-Abu, His-Pro-Phe-Ada, His-Pro-Phe-Arg, His-Pro-Phe-Asn, His-Pro-Phe-Bia, His-Pro-Phe-Dab, His-Pro-Phe-Gln, His-Pro-Phe-Gly, His-Pro-Phe(N-im-Alkyl-His), His-Pro-Phe-Ile, His-Pro-Phe-Leu, His-Pro-Phe-tert.-Leu, His-Pro-Phe-Lys, His-Pro-Phe-Met, His-Pro-Phe-Nbg, His-Pro-Phe-Nle, His-Pro-Phe-(N-Me-His), His-Pro-Phe(N-Me-Phe), His-Pro-Phe-Orn, His-Pro-Phe-Pro, His-Pro-Phe-Ser, His-Pro-Phe-Thr, His-Pro-Phe-Tic, His-Pro-Phe-Trp, His-Pro-Phe-Tyr, His-Pro-Phe-Val, His-Pro-Pro-His, His-Pro-Ser-His, His,Pro-Thr-His, His-Pro-Tic-His, His-Pro-Trp-His, His-Pro-Tyr-His, His-Pro-Val-His.

E bedeutet vorzugsweise OH, OCH₃, OC₂H₅, NH₂, NHCH₃ oder N(CH₃)₂.

Die Gruppe W bedeutet vorzugsweise -NH-CHR³-CHOH-CH₂-CO-, insbesondere -NH-CH-(Cyclohexylmethyl)-CHOH-CH₂-CO-("AHCP", abgeleitet von 4-Amino-3-hydroxy-5-cyclohexylpentansäure), oder -NH-CH(CH₂CH₂-Cyclohexyl)-CHOH-CH₂-CO-("AHCH"; abgeleitet von 4-Amino-3-hydroxy-6-cyclohexyl-hexansäure).

Die Gruppe W besitzt mindestens zwei chirale Zentren. Die Verbindungen der Formel I können daher in verschiedenen -optisch-inaktiven oder optisch-aktiven-Formen vorkommen. Die Formel I umschließt alle diese Formen. Falls W -NH-CHR³-CHOH-CH₂-CO-bedeutet, sind die 3S-Hydroxy-4S-amino-Enantiomeren bevorzugt. Wenn bei der Bezeichnung von Einzelsubstanzen nichts anderes angegeben ist, beziehen sich die Abkürzungen AHCP und AHCH immer auf diese 3S,4S-Formen.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln Ia bis If ausgedrückt werden, die der Formel I entsprechen, worin jedoch

in Ia X H, POA, BOC, 4-Phenylbutyryl, 2-Benzyl-3-phenylpropionyl, 2-Benzyl-4-phenylbutyryl, 2-(2-Phenylethyl)-4-phenylbutyryl, 2-(2-Naphthylmethyl)-4-phenylbutyryl, oder CBZ,

Z His, Ada-His, Cal-His, Nle-His, Phe-Abu, Phe-Dab, Phe-His, Phe-Lys, Phe-Met, Phe-(N-im-methyl)-His, Phe-Nle, Phe-Orn, Pro-Phe-His oder His-Pro-Phe-His,

$R^2$ und $R^4$ jeweils H,

$R^3$ Cyclohexylmethyl oder 2-Cyclohexylethyl,

E OH, OMe, OEt, $NH_2$, $NH(CH_3)$ oder $N(CH_3)_2$ und

n 1 bedeuten;

in Ib X H, BOC, 2-Benzyl-3-phenylpropionyl, 2-Benzyl-4-phenylbutyryl, 2-(2-Phenylethyl)-4-phenylbutyryl oder 2-(2-Naphthylmethyl)-4-phenylbutyryl,

Z His, Ada-His, Cal-His, Nle-His, Phe-Abu, Phe-Dab, Phe-His, Phe-Lys, Phe-Met, Phe-(N-im-methyl)-His, Phe-Nle oder Phe-Orn,

$R^2$ und $R^4$ jeweils H,

$R^3$ Cyclohexylmethyl,

E OH, OMe, OEt, $NH_2$, $NH(CH_3)$ oder $N(CH_3)_2$ und

n 1 bedeuten;

in Ic X H, BOC oder 2-Benzyl-4-phenylbutyryl,

Z His, Phe-Nle oder Phe-His,

$R^2$ und $R^4$ jeweils H,

$R^3$ Cyclohexylmethyl,

E OH, OMe, OEt, $NH_2$, $NH(CH_3)$ oder $N(CH_3)_2$ und

n 1 bedeuten;

in Id X BOC oder 2-Benzyl-4-phenylbutyryl,

Z His, Phe-Nle oder Phe-His,

$R^2$ und $R^4$ jeweils H,

$R^3$ Cyclohexylmethyl,

E OH, OMe, $NH_2$ oder $N(CH_3)_2$ und

n 1 bedeuten;

in Ie X BOC,

Z Phe-Nle oder Phe-His,

$R^2$ und $R^4$ jeweils H,

$R^3$ Cyclohexylmethyl,

E OH oder OMe und

n 1 bedeuten;

in If X Pyrrolidinocarbonyl, Piperidinocarbonyl, Morpholinocarbonyl oder Thiomorpholinocarbonyl bedeutet.

Gegenstand der Erfindung ist ferner ein Verfahren zur Herstellung eines Peptids der Formel I sowie von seinen Salzen, dadurch gekennzeichnet, daß man es aus einem seiner funktionellen Derivate durch Behandeln mit einem solvolysierenden oder hydrogenolysierenden Mittel in Freiheit setzt

oder daß man eine Verbindung, die der Formel I entspricht, aber an Stelle von H-Atomen eine oder mehrere zusätzliche C-C-und/oder C-N-und/oder C-O-Bindungen enthält, reduziert

oder daß man eine Carbonsäure der Formel II

X-G$^1$-OH II

worin G$^1$ (a) Z$^1$,

(b) Z bedeutet

mit einer Aminoverbindung der Formel III

H-G$^2$ III

worin

G$^2$ (a) Z$^2$-W-E,

(b) W-E,

W -$NR^2$-$CHR^3$-CHOH-$(CHR^4)_n$-CO-und

Z$^1$ + Z$^2$ zusammen Z bedeuten

umsetzt,

und daß man gegebenenfalls in einer Verbindung der Formel I eine funktionell abgewandelte Amino-und/oder Hydroxygruppe durch Behandeln mit solvolysierenden oder hydrogenolysierenden Mitteln in Freiheit setzt und/oder einen Rest E durch Behandeln mit veresternden, solvolysierenden oder amidierenden Mitteln in einen anderen Rest E umwandelt und/oder eine Verbindung der Formel I durch Behandeln mit einer Säure oder Base in eines ihrer Salze überführt.

Die Verbindungen der Formel I und auch die Ausgangsstoffe zu ihrer Herstellung werden im übrigen nach an sich bekannten Methoden hergestellt, wie sie in der Literatur (z. B. in den Standardwerken wie Houben-Weyl, Methoden der Organischen Chemie, Georg-Thieme-Verlag, Stuttgart; ferner EP-A-45665, EP-A-77028, EP-A-77029, EP-A-81783) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.

Vorzugsweise werden die Verbindungen der Formel I erhalten, indem man sie aus ihren funktionellen Derivaten durch Solvolyse, insbesondere Hydrolyse, oder durch Hydrogenolyse in Freiheit setzt.

Bevorzugte Ausgangsstoffe für die Solvolyse bzw. Hydrogenolyse sind solche, die an Stelle einer oder mehrerer freier Amino-und/oder Hydroxygruppen entsprechende geschützte Amino-und/oder Hydroxygruppen enthalten, vorzugsweise solche, die an Stelle eines H-Atoms, das mit einem N-Atom verbunden ist, eine Aminoschutzgruppe tragen, z. B. solche, die der Formel I entsprechen, aber an Stelle einer His-Gruppe eine N(im)-R⁸-His-Gruppe (worin R⁸ eine Aminoschutzgruppe bedeutet, z. B. BOM oder DNP) enthalten.

Ferner sind Ausgangsstoffe bevorzugt, die an Stelle des H-Atoms einer Hydroxygruppe eine Hydroxyschutzgruppe tragen, z. B. solche der Formel X-Z-NR²-CHR³-CHOR⁶-(CHR⁴)ₙ-CO-E, worin R⁶ eine Hydroxyschutzgruppe bedeutet.

Es können auch mehrere -gleiche oder verschiedene -geschützte Amino-und/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Amino gruppe vor chemischen Umsetzungen zu schützen (zu blockieren), die aber leicht entfernbar sind, nachdem die gewünschte chemische Reaktion an anderen Stellen des Moleküls durchgeführt worden ist. Typisch für solche Gruppen sind insbesondere unsubstituierte oder substituierte Acyl-, Aryl-(z. B. 2,4-Dinitrophenyl), Aralkoxymethyl-(z. B. Benzyloxymethyl) oder Aralkylgruppen (z. B. Benzyl, 4-Nitrobenzyl, Triphenylmethyl). Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren in weitestem Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl-und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie Phenoxyacetyl; Alkoxycarbonyl wie Methoxycarbonyl, Ethoxycarbonyl, 2,2,2-Trichlorethoxycarbonyl, BOC, 2-Jodethoxycarbonyl; Aralkyloxycarbonyl wie CBZ ("Carbobenzoxy"), 4-Methoxybenzyloxycarbonyl, FMOC. Bevorzugte Aminoschutzgruppen sind DNP, BOM, CBZ, FMOC, Benzyl und Acetyl.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an anderen Stellen des Moleküls durchgeführt worden ist. Typisch für solche Gruppen sind die oben genannten unsubstituierten oder substituierten Aryl-, Aralkyl-oder Acylgruppen, ferner auch Alkylgruppen. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. Benzyl, p-Nitrobenzoyl, p-Toluolsulfonyl und Acetyl, wobei Benzyl und Acetyl besonders bevorzugt sind.

Die Hydroxygruppe kann auch Teil einer Carboxygruppe sein, so daß als Hydroxyschutzgruppe eine Carboxyschutzgruppe verwendet wird. So kann die Carboxygruppe nach dem Prinzip der "Merrifield"-Synthese an ein Polymeres gebunden sein, z. B. in Esterform.

Die als Ausgangsstoffe zu verwendenden funktionellen Derivate der Verbindungen der Formel I können nach üblichen Methoden der Aminosäure-und Peptidsynthese hergestellt werden, wie sie z. B. in den genannten Standardwerken und Patentanmeldungen beschrieben sind.

Das In-Freiheit-Setzen der Verbindungen der Formel I aus ihren funktionellen Derivaten gelingt -je nach der benutzten Schutzgruppe -z. B. mit starken Säuren, zweckmäßig mit Trifluoressigsäure oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich. Als inerte Lösungsmittel eignen sich vorzugsweise organische, beispielsweise Carbonsäuren wie Essigsäure, Ether wie Tetrahydrofuran oder Dioxan, Amide wie Dimethylformamid (DMF), halogenierte Kohlenwasserstoffe wie Dichlormethan, ferner auch Alkohole wie Methanol, Ethanol oder Isopropanol sowie Wasser. Ferner kommen Gemische der vorgenannten Lösungsmittel in Frage. Trifluor essigsäure wird vorzugsweise im Überschuß ohne Zusatz eines weiteren Lösungsmittels verwendet, Perchlorsäure in Form eines Gemisches aus Essigsäure und 70 %iger Perchlorsäure im Verhältnis 9 : 1. Die Reaktionstemperaturen für die Spaltung liegen zweckmäßig zwischen etwa 0 und etwa 50°, vorzugsweise arbeitet man zwischen 15 und 30° - (Raumtemperatur).

Die BOC-Gruppe kann z. B. bevorzugt mit 40 %iger Trifluoressigsäure in Methylenchlorid oder mit etwa 3 bis 5 n HCl in Dioxan bei 15-30° abgespalten werden, die FMOC-Gruppe mit einer etwa 5-bis 20%igen Lösung von Dimethylamin, Diethylamin oder Piperidin in DMF bei 15-30°. Eine Abspaltung der DNP-Gruppe gelingt z. B. auch mit einer etwa 3-bis 10%igen Lösung von 2-Mercaptoethanol in DMF/Wasser bei 15-30°. Nach der "Merrifield"-Methode werden Verbindungen der Formel I (E = OH) zweckmäßig mit Trifluoressigsäure von dem polymeren Träger abgespalten.

Hydrogenolytisch entfernbare Schutzgruppen - (z. B. BOM, CBZ oder Benzyl) können z. B. durch Behandeln mit Wasserstoff in Gegenwart eines Katalysators (z. B. eines Edelmetallkatalysators wie Palladium, zweckmäßig auf einem Träger wie Kohle) abgespalten werden. Als Lösungsmittel eignen sich dabei die oben angegebenen, insbesondere z. B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt. Eine Hydrogenolyse der CBZ-Gruppe gelingt z. B. gut an 5-bis 10%igem Pd-C in Methanol bei 20-30°.

Die Verbindungen der Formel I sind auch erhältlich durch Reduktion entsprechender Verbindungen, die an Stelle von H-Atomen eine oder mehrere zusätzliche C-C-und/oder C-N-und/oder C-O-Bindungen enthalten.

So können z. B. Ketoverbindungen der Formel IV

$$X\text{-}Z\text{-}NR^2\text{-}CHR^3\text{-}CO\text{-}(CHR^5)_n\text{-}CO\text{-}E \quad IV$$

zu Verbindungen der Formel I reduziert werden, beispielsweise mit einem komplexen Metallhydrid wie $NaBH_4$, das nicht gleichzeitig die Peptid-Carbonylgruppen reduziert, in einem inerten Lösungsmittel wie Methanol bei Temperaturen zwischen etwa -10 und +30°. Die Verbindungen der Formel IV sind z. B. erhältlich durch Reaktion einer Aminosäure der Formel $X\text{-}Z\text{-}NR^2\text{-}CHR^3\text{-}COOH$ mit Carbonyldiimidazol zum entsprechenden Imidazolid und anschließende Umsetzung mit Malonsäurederivaten der Formel $HOOC\text{-}CH_2\text{-}CO\text{-}E$ oder deren Estern oder Salzen sowie nachfolgende Decarboxylierung.

Verbindungen der Formel I können auch durch direkte Peptidsynthese aus einer Carbonsäure-und einer Aminkomponente erhalten werden. Als Carbonsäurekomponenten eignen sich z. B. solche der Teilformel X-Z-OH, als Aminkomponenten solche der Teilformel H-W-E. Die Peptidbindung kann aber auch innerhalb der Gruppe Z geknüpft werden; dabei wird eine Carbonsäure der Formel X-Z¹-OH mit einem Aminopeptid der Formel H-Z²-W-E umgesetzt, wobei Z¹ + Z² = Z ist. Dabei arbeitet man zweckmäßig nach üblichen Methoden der Peptid-Synthese, wie sie z. B. in Houben-Weyl, 1.c., Band 15/II, Seiten 1 bis 806 (1974) beschrieben sind.

Die Reaktion gelingt vorzugsweise in Gegenwart eines Dehydratisierungsmittels, z. B. eines Carbodiimids wie DCCI oder Dimethylaminopropylethyl-carbodiimid, ferner Propanphosphonsäureanhydrid (vgl. Angew.Chem. 92, 129 (1980)), Diphenylphosphorylazid oder 2-Ethoxy-N-ethoxycarbonyl-1,2-dihydrochinolin, in einem inerten Lösungsmittel, z. B. einem halogenierten Kohlenwasserstoff wie Dichlormethan, einem Ether wie Tetrahydrofuran oder Dioxan, einem Amid wie DMF oder Dimethylacetamid, einem Nitril wie Acetonitril, bei Temperaturen zwischen etwa -10 und 40, vorzugsweise zwischen 0 und 30°.

An Stelle von II bzw. III können auch geeignete reaktionsfähige Derivate dieser Stoffe in die Reaktion eingesetzt werden, z. B. solche, in denen reaktive Gruppen intermediär durch Schutzgruppen blockiert sind. Die Aminosäurederivate III können z. B. in Form ihrer aktivierten Ester verwendet werden, die zweckmäßig in situ gebildet werden, z. B. durch Zusatz von 1-Hydroxybenztriazol oder N-Hydroxysuccinimid.

Die Ausgangsstoffe der Formeln II und III sind großenteils bekannt. Sofern sie nicht bekannt sind, können sie nach bekannten Methoden, z. B. den oben angegebenen Methoden der Peptidsynthese und der Abspaltung von Schutzgruppen, hergestellt werden.

Gewünschtenfalls kann in einer Verbindung der Formel I eine funktionell abgewandelte Amino-und/oder Hydroxygruppe durch Solvolyse oder Hydrogenolyse nach einer der oben beschriebenen Methoden in Freiheit gesetzt werden.

So kann insbesondere eine Verbindung der Formel I, worin X von H verschieden ist, in eine Verbindung der Formel I (X = H) umgewandelt werden, zweckmäßig durch Hydrogenolyse, falls X = CBZ ist, sonst durch selektive Solvolyse. Falls X = BOC ist, kann man z. B. mit HCl in Dioxan bei Raumtemperatur die BOC-Gruppe abspalten.

Weiterhin ist es möglich, einen Rest E durch Behandeln mit veresternden, solvolysierenden oder amidierenden Mitteln in einen anderen Rest E umzuwandeln. So kann man eine Säure der Formel I - (E = OH) verestern, z. B. mit Hilfe eines Alkohols der Formel A-OH oder eines Diazoalkans, z. B. Diazomethan, oder man kann einen Ester der Formel I (E = OA) zur entsprechenden Säure der Formel I (E = OH) verseifen, Z. B. mit wässerig-dioxanischer Natronlauge bei Raumtemperatur. Ferner kann man z. B. einen Ester der Formel I (E = OA) durch Behandeln mit Ammoniak oder mit einem Amin der Formel A-$NH_2$ oder $A_2$NH in das entsprechende Amid der Formel I (E = $NH_2$, NHA oder $NA_2$) überführen.

Eine Base der Formel I kann mit einer Säure in das zugehörige. Säureadditionssalz übergeführt werden. Für diese Umsetzung kommen insbesondere Säuren in Frage, die physiologisch unbedenkliche Salze liefern. So können anorganische Säuren verwendet werden, z. B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein-oder mehrbasige Carbon-, Sulfon-oder Schwefelsäuren, z. B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2-oder 3-Phenylpropionsäure, Citronensäure, Gluconsäure, Ascorbinsäure, Nicotinsäure, Isonicotinsäure, Methan-oder Ethansulfonsäure, Ethandisulfonsäure, 2-Hydroxyethansulfonsäure, Benzolsulfonsäure, p-Toluolsulfonsäure, Naphthalin-mono-und-disulfonsäuren, Laurylschwefelsäure. Salze mit physiologisch nicht unbedenklichen Säuren, z. B. Pikrate, können zur Isolierung und/oder Aufreinigung der Verbindungen der Formel I verwendet werden.

Eine Säure der Formel I kann durch Umsetzung mit einer Base in eines ihrer physiologisch unbedenklichen Metall-bzw. Ammoniumsalze übergeführt werden. Als Salze kommen insbesondere die Natrium-, Kalium-, Magnesium-, Calcium-und Ammoniumsalze in Betracht, ferner substituierte Ammoniumsalze, z. B. die Dimethyl-, Diethyl-oder Diisopropylammonium-, Monoethanol-, Diethanol-und Triethanolammonium, Cyclohexylammonium-, Dicyclohexylammonium-und Dibenzylethylendiammoniumsalze, weiterhin z. B. Salze mit N-Methyl-D-glucamin oder mit basischen Aminosäuren wie Arginin oder Lysin.

Die neuen Verbindungen der Formel I und ihre physiologisch unbedenklichen Salze können zur Herstellung pharmazeutischer Präparate verwendet werden, indem man sie zusammen mit mindestens einem Träger-oder Hilfsstoff und, falls erwünscht, zusammen mit einem oder mehreren weiteren Wirkstoff(en) in eine geeignete Dosierungsform bringt. Die so erhaltenen Zubereitungen können als Arzneimittel in der Human-oder Veterinärmedizin eingesetzt werden. Als Trägersubstanzen kommen organische oder anorganische Stoffe in Frage, die sich für die enterale (z. B. orale oder rektale) oder parenterale Applikation oder für eine Applikation in Form eines Inhalations-Sprays eignen und mit den neuen Verbindungen nicht reagieren, beispielsweise Wasser, pflanzliche Öle, Benzylalkohole, Polyethylenglykole, Glycerintriacetat und andere Fettsäureglyceride, Gela tine, Sojalecithin, Kohlehydrate wie Lactose oder Stärke, Magnesiumstearat, Talk, Cellulose. Zur oralen Anwendung dienen insbesondere Tabletten, Dragees, Kapseln, Sirupe, Säfte oder Tropfen; von Interesse sind speziell ·Tabletten und Kapseln mit magensaftresistenten Überzügen bzw. Kapselhüllen. Zur rektalen Anwendung dienen Suppositorien, zur parenteralen Applikation Lösungen, vorzugsweise ölige oder wässerige Lösungen, ferner Suspensionen, Emulsionen oder Implantate. Für die Applikation als Inhalations-Spray können Sprays verwendet werden, die den Wirkstoff entweder gelöst oder suspendiert in einem Treibgasgemisch (z. B. Fluorchlorkohlenwasserstoffen) enthalten. Zweckmäßig verwendet man den Wirkstoff dabei in mikronisierter Form, wobei ein oder mehrere zusätzliche physiologisch verträgliche Lösungsmittel zugegen sein können, z. B. Ethanol. Inhalationslösungen können mit Hilfe üblicher Inhalatoren verabfolgt werden. Die neuen Verbindungen können auch lyophilisiert und die erhaltenen Lyophilisate z. B. zur Herstellung von Injektionspräparaten verwendet werden. Die angegebenen Zubereitungen können sterilisiert sein und/oder Hilfsstoffe wie Konservierungs-, Stabilisierungs-und/oder Netzmittel, Emulgatoren, Salze zur Beeinflussung des osmotischen Druckes, Puffersubstanzen, Farb-und/oder Aromastoffe enthalten. Sie können, falls erwünscht, auch einen oder mehrere weitere Wirkstoffe enthalten, Z. B. ein oder mehrere Vitamine.

Die erfindungsgemäßen Substanzen werden in der Regel in Analogie zu anderen bekannten, im Handel befindlichen Peptiden, insbesondere aber in Analogie zu den in der EP-A-77028 beschriebenen Verbindungen verabreicht, vorzugsweise in Dosierungen zwischen etwa 100 mg und 30 g, insbesondere zwischen 500 mg und 5 g pro Dosierungseinheit. Die tägliche Dosierung liegt vorzugsweise zwischen etwa 2 und 600 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die parenterale Applikation ist bevorzugt.

Vor-und nachstehend sind alle Temperaturen in °C angegeben. In den nachfolgenden Beispielen bedeutet "übliche Aufarbeitung": Man gibt, falls erforderlich, Wasser hinzu, stellt durch Zugabe von HCl oder NaOH auf den angegebenen pH-Wert ein, extrahiert mit Ether oder Dichlormethan, trennt ab, trocknet die organische Phase über Natriumsulfat, filtriert, dampft ein und reinigt durch Chromatographie an Kieselgel und/oder Kristallisation.

Beispiel 1

Ein Gemisch von 1 g 4S-[N-tert.-Butoxycarbonyl-L-phenylalanyl-N(imi)-(2,4-dinitrophenyl)-L-histidyl]-3S-hydroxy-5-cyclohexylpentansäure ["BOC-Phe-(imi-DNP-His)-AHCP-OH"; erhältlich durch Reaktion von AHCP-OMe mit BOD-(imi-DNP-His)-OH zu BOC-(imi-DNP-His)-AHCP-OMe, Hydrolyse zu H-(imi-DNP-His)-AHCP-OMe, Reaktion mit BOC-Phe-OH zu BOC-Phe-(imi-DNP-His)-AHCP-OMe und Verseifung mit NaOH in Dioxan/Wasser], 2 g 2-Mercaptoethanol, 25 ml DMF und 25 ml Wasser wird unter Rühren bei 20° auf pH 8 eingestellt und 2 Std. bei 20° gerührt. Nach üblicher Aufarbeitung (pH 3,5) erhält man 4S-(N-tert.-Butoxycarbonyl-L-phenylalanyl-L-histidyl)-3S-hydroxy-5-cyclohexylpentansäure ("BOC-Phe-His-AHCP-OH"), F. 206° (Zers.).

Analog erhält man aus den entsprechenden imi-DNP-His-Derivaten:

Acetyl-Phe-His-AHCP-OH

Isobutyryl-Phe-His-AHCP-OH

Isovaleryl-Phe-His-AHCP-OH

Benzoyl-Phe-His-AHCP-OH

Phenylacetyl-Phe-His-AHCP-OH

α-Naphthylacetyl-Phe-His-AHCP-OH

3-Phenylpropionyl-Phe-His-AHCP-OH

3-p-Tolylpropionyl-Phe-His-AHCP-OH

3-o-Methoxyphenylpropionyl-Phe-His-AHCP-OH

3-p-Methoxyphenylpropionyl-Phe-His-AHCP-OH

3-p-Fluorphenylpropionyl-Phe-His-AHCP-OH

3-p-Chlorphenylpropionyl-Phe-His-AHCP-OH

3-p-Bromphenylpropionyl-Phe-His-AHCP-OH

3-p-Jodphenylpropionyl-Phe-His-AHCP-OH

3-m-Trifluormethylphenylpropionyl-Phe-His-AHCP-OH

Cyclopropylcarbonyl-Phe-His-AHCP-OH

Cyclopentylcarbonyl-Phe-His-AHCP-OH

Cyclohexylcarbonyl-Phe-His-AHCP-OH

3-Cyclohexylpropionyl-Phe-His-AHCP-OH

6-Cycloheptylhexanoyl-Phe-His-AHCP-OH

POA-Phe-His-AHCP-OH

CBZ-Ada-His-AHCP-OH

CBZ-Cal-His-AHCP-OH

CBZ-αNal-His-AHCP-OH

CBZ-βNal-His-AHCP-OH

CBZ-Phe-His-AHCP-OH

CBZ-Trp-His-AHCP-OH

CBZ-Tyr-His-AHCP-OH

2-Benzyl-3-phenylpropionyl-Phe-His-AHCP-OH

4-Phenylbutyryl-Phe-His-AHCP-OH

2-Benzyl-4-phenylbutyryl-Phe-His-AHCP-OH

2-(2-Phenylethyl)-4-phenylbutyryl-Phe-His-AHCP-OH

2-(2-Naphthylmethyl)-4-phenylbutyryl-Phe-His-AHCP-OH

POA-Phe-His-AHCP-OH

CBZ-Phe-His-AHCH-OH.


Beispiel 2

Man löst 1 g BOC-Phe(imi-BOM-His)-AHCP-OH [F. 198°; erhältlich durch Reaktion von AHCP-OMe mit BOC-(imi-BOM-His)-OH zu BOC-(imi-BOM-His)-AHCP-OMe (F. 116°), Hydrolyse zu H-(imi-BOM-His)-AHCP-OMe (Hydrochlorid, F. 123°), Reaktion mit BOC-Phe-OH zu BOC-Phe-(imi-BOM-His)-AHCP-OMe (F. 74°) und Verseifung mit NaOH in wässerigem Dioxan] in 10 ml Methanol, hydriert an 0,5 g 5%ig. Pd-C bei 20° und 1 bar, filtriert, dampft ein und erhält BOC-Phe-His-AHCP-OH, F. 206° (Zers.).

Die gleiche Substanz ist aus dem gleichen Ausgangsstoff auch erhältlich durch Behandeln mit Ammoniumformiat/10%ig. Pd-C in Methanol bei 20°.

Analog erhält man aus den entsprechenden imi-BOM-Derivaten:

BOC-His-AHCP-OH

BOC-Phe-His-AHCP-OMe

BOC-Phe-His-AHCP-OEt

BOC-Phe-His-AHCP-NH$_2$, F. 198° (Zers.)

BOC-Phe-His-AHCP-NHCH$_3$

BOC-Phe-His-AHCP-N(CH$_3$)$_2$, F. 189° (Zers.)

BOC-Abu-His-AHCP-OH

BOC-Ada-His-AHCP-OH

BOC-Ala-His-AHCP-OH

BOC-Arg-His-AHCP-OH

BOC-Asn-His-AHCP-OH

BOC-Bia-His-AHCP-OH

BOC-Cal-His-AHCP-OH, F. 152-154°

BOC-Dab-His-AHCP-OH

BOC-Gln-His-AHCP-OH

BOC-Gly-His-AHCP-OH

BOC-His-His-AHCP-OH

BOC-N(im)-Methyl-His-His-AHCP-OH

BOC-Ile-His-AHCP-OH

BOC-Leu-His-AHCP-OH

BOC-tert.-Leu-His-AHCP-OH

BOC-Lys-His-AHCP-OH

BOC-Met-His-AHCP-OH

BOC-αNal-His-AHCP-OH, F. 163-165°

BOC-βNal-His-AHCP-OH, F. 187°

[aus BOC-β-Nal-(imi-BOM-His)-AHCP-OMe (F. 92-94°) über BOC-β-Nal-(imi-BOM-His)-AHCP-OH (F. 174-176°)]

BOC-Nbg-His-AHCP-OH

BOC-Nle-His-AHCP-OH

BOC-Orn-His-AHCP-OH

BOC-Phe-His-AHCP-OH

BOC-Pro-His-AHCP-OH

BOC-Ser-His-AHCP-OH

BOC-Thr-His-AHCP-OH

BOC-Tic-His-AHCP-OH

BOC-Trp-His-AHCP-OH

BOC-Tyr-His-AHCP-OH

BOC-Val-His-AHCP-OH

(2-Benzyl-4-phenylbutyryl-His)-AHCP-OH, F. 191° - [Zers.; erhältlich aus H-(imi-BOM-His)-AHCP-OMe über (2-Benzyl-4-phenylbutyryl-imi-BOM-His)-AHCP-OMe (F. 125-128°) und (2-Benzyl-4-phenylbutyryl-imi-BOM-His)-AHCP-OH (F. 184°)]

[2-(2-Phenylethyl)-4-phenylbutyryl-His]-AHCP-OH

(2-Benzyl-3-phenylpropionyl-His)-AHCP-OH

[2-(2-Naphthylmethyl)-4-phenylbutyryl-His]-AHCP-OH

(2-Benzyl-4-phenylbutyryl-His)-AHCP-N(CH$_3$)$_2$, F. 73-75°

POA-His-AHCP-OH

BOC-Phe-His-AHCH-OH

BOC-Phe-His-AHCH-OMe

BOC-Phe-His-AHCH-OEt

BOC-Phe-His-AHCH-NH$_2$

BOC-Phe-His-AHCH-NHCH$_3$

BOC-Phe-His-AHCH-N(CH$_3$)$_2$

N-Ethylcarbamoyl-Phe-His-AHCP-NHCH$_2$CH$_2$CH-(CH$_3$)$_2$

N-Isopropylcarbamoyl-Phe-His-AHCP-NHCH$_2$CH$_2$CH(CH$_3$)$_2$

N-Isopropylcarbamoyl-Phe-His-AHCP-NHCH$_2$CH-(CH$_3$)C$_2$H$_5$

N-Isopropylcarbamoyl-Phe-His-AHCP-N(CH$_3$)$_2$

N-Isopropylcarbamoyl-Phe-His-AHCP-OH

Morpholinocarbonyl-Phe-His-AHCP-OH, F. 150° - (Zers.)

Morpholinocarbonyl-Phe-His-AHCP-NHCH$_2$CH$_2$(CH-(CH$_3$)$_2$

Morpholinocarbonyl-Phe-His-AHCP-NHCH$_2$CH-(CH$_3$)C$_2$H$_5$,

F. 110-112°.

Beispiel 3

Eine Lösung von 2,66 g AHCP-Methylester-hydrochlorid in 50 ml Dichlormethan wird mit 1,01 g N-Methylmorpholin versetzt. Man gibt 3,78 g BOC-Phe-Nle-OH, 1,35 g HOBt und eine Lösung von 2,06 g DCCI in 50 ml Dichlormethan hinzu, rührt 14 Std. bei 4°, filtriert den ausgefallenen Dicyclohexylharnstoff ab, dampft ein und arbeitet wie üblich auf (pH 8). Man erhält BOC-Phe-Nle-AHCP-OMe, F. 179°.

Analog erhält man mit den entsprechenden BOC-Peptiden:

BOC-Phe-Abu-AHCP-OMe    BOC-Phe-Ada-AHCP-OMe

BOC-Phe-Ala-AHCP-OMe    BOC-Phe-Arg-AHCP-OMe

BOC-Phe-Asn-AHCP-OMe OMe, F. 95-98°    BOC-Phe-Bia-AHCP-OMe

BOC-Phe-Cal-AHCP-OMe OMe    BOC-Phe-Dab-AHCP-OMe

BOC-Phe-Gln-AHCP-OMe OMe, F. 73-76°    BOC-Phe-Gly-AHCP-OMe

BOC-Phe-His-AHCP-OMe    BOC-Phe-N(im)-methyl-His-AHCP-OMe

BOC-Phe-Ile-AHCP-OMe OMe    BOC-Phe-Leu-AHCP-OMe

BOC-Phe-tert.-Leu-AHCP-OMe

BOC-Phe-Lys-AHCP-OMe

BOC-Phe-Met-AHCP-OMe

BOC-Phe-αNal-AHCP-OMe

BOC-Phe-βNal-AHCP-OMe

BOC-Phe-Nbg-AHCP-OMe

BOC-Phe-Orn-AHCP-OMe

BOC-Phe-Phe-AHCP-OMe

BOC-Phe-Pro-AHCP-OMe

BOC-Phe-Ser-AHCP-OMe

BOC-Phe-Thr-AHCP-OMe

BOC-Phe-Tic-AHCP-OMe

BOC-Phe-Trp-AHCP-OMe

BOC-Phe-Tyr-AHCP-OMe

BOC-Phe-Val-AHCP-OMe

BOC-Abu-His-AHCP-OMe

BOC-Ada-His-AHCP-OMe

BOC-Ala-His-AHCP-OMe

BOC-Arg-His-AHCP-OMe

BOC-Asn-His-AHCP-OMe

BOC-Bia-His-AHCP-OMe

BOC-Cal-His-AHCP-OMe

BOC-Dab-His-AHCP-OMe

BOC-Gln-His-AHCP-OMe

BOC-Gly-His-AHCP-OMe

BOC-His-His-AHCP-OMe

BOC-N(im)-Methyl-His-His-AHCP-OMe

BOC-Ile-His-AHCP-OMe

BOC-Leu-His-AHCP-OMe

BOC-tert.Leu-His-AHCP-OMe

BOC-Lys-His-AHCP-OMe

BOC-Met-His-AHCP-OMe

BOC-αNal-His-AHCP-OMe

BOC-βNal-His-AHCP-OMe

BOC-Nbg-His-AHCP-OMe

BOC-Nle-His-AHCP-OMe

BOC-Orn-His-AHCP-OMe        BOC-Phe-His-AHCP-OMe

BOC-Pro-His-AHCP-OMe  BOC-Ser-His-AHCP-OMe

BOC-Thr-His-AHCP-OMe  BOC-Tic-His-AHCP-OMe

BOC-Trp-His-AHCP-OMe  BOC-Tyr-His-AHCP-OMe

BOC-Val-His-AHCP-OMe

BOC-Phe-Gly-AHCP-NH-CH$_2$CH(CH$_3$)$_2$, F. 84-86°

BOC-Phe-Gly-AHCP-NH-CH$_2$CH$_2$(CH$_3$)$_2$, F. 87-89°

BOC-Phe-Gly-AHCP-NH-CH$_2$CH(CH$_3$)C$_2$H$_5$

Morpholinocarbonyl-Phe-Gly-AHCP-OH, F. 88-90°

Morpholinocarboyl-Phe-Gly-AHCP-NH-CH$_2$CH(CH$_3$)$_2$

Morpholinocarbonyl-Phe-Gly-AHCP-NH-CH$_2$CH$_2$CH-(CH$_3$)$_2$

Morpholinocarbonyl-Phe-Gly-AHCP-NH-CH$_2$CH-(CH$_3$)C$_2$H$_5$,

F. 116-118°.

Beispiel 4

Analog Beispiel 3 erhält man aus BOC-Phe-OH und H-Nle-AHCP-NH$_2$-hydrochlorid/N-Methylmorpholin/HOBt/DCCI das BOC-Phe-Nle-AHCP-NH$_2$.

Analog erhält man mit H-Nle-ACHP-OMe -(herstellbar aus BOC-Nle-AHC-OMe, Rf 0,9 an Kieselgel, CH$_2$Cl$_2$/CH$_3$OH 9:1) das BOC-Phe-Nle-AHCP-OMe, F. 179°.

Beispiel 5

Analog Beispiel 3 erhält man aus BOC-Phe-Met-AHCP-OH und Dimethylaminhydrochlorid/N-Methylmorpholin/HOBt/DCCI das BOC-Phe-Met-AHCP-N(CH$_3$)$_2$.

Beispiel 6

Eine Lösung von 1 g BOC-Phe-His AHCP-OH in 20 ml 4 n HCl in Dioxan wird 30 Min. bei 20° gerührt und dann eingedampft. Nach üblicher Aufarbeitung erhält man H-Phe-His-AHCP-OH.

Analog erhält man durch Spaltung der entsprechenden BOC-Derivate:

H-Phe-His-AHCP-OMe

H-Phe-His-AHCP-OEt

H-Phe-His-AHCP-NH$_2$

H-Phe-His-AHCP-NHCH$_3$

H-Phe-His-AHCP-N(CH$_3$)$_2$
H-Abu-His-AHCP-OH

H-Ada-His-AHCP-OH

H-Ala-His-AHCP-OH

H-Arg-His-AHCP-OH

H-Asn-His-AHCP-OH

H-Bia-His-AHCP-OH

H-Cal-His-AHCP-OH

H-Dab-His-AHCP-OH

H-Gln-His-AHCP-OH

H-Gly-His-AHCP-OH

H-His-His-AHCP-OH

H-N(im)-Methyl-His-His-AHCP-OH

H-Ile-His-AHCP-OH

H-Leu-His-AHCP-OH

H-tert.-Leu-His-AHCP-OH

H-Lys-His-AHCP-OH

H-Met-His-AHCP-OH

H-αNal-His-AHCP-OH

H-βNal-His-AHCP-OH

H-Nbg-His-AHCP-OH

H-Nle-His-AHCP-OH

H-Orn-His-AHCP-OH

H-Phe-His-AHCP-OH

H-Pro-His-AHCP-OH

H-Ser-His-AHCP-OH

H-Thr-His-AHCP-OH

H-Tic-His-AHCP-OH

H-Trp-His-AHCP-OH

H-Tyr-His-AHCP-OH

H-Val-His-AHCP-OH

H-Phe-Abu-AHCP-OH

H-Phe-Ada-AHCP-OH

H-Phe-Ala-AHCP-OH

H-Phe-Arg-AHCP-OH

H-Phe-Asn-AHCP-OH

H-Phe-Bia-AHCP-OH

H-Phe-Cal-AHCP-OH

H-Phe-Dab-AHCP-OH

H-Phe-Gln-AHCP-OH

H-Phe-Gly-AHCP-OH

H-Phe-N(im)-methyl-His-AHCP-OH

H-Phe-Ile-AHCP-OH

H-Phe-Leu-AHCP-OH

H-Phe-tert.-Leu-AHCP-OH

H-Phe-Lys-AHCP-OH

H-Phe-Met-AHCP-OH

H-Phe-αNal-AHCP-OH

H-Phe-βNal-AHCP-OH

H-Phe-Nbg-AHCP-OH

H-Phe-Nle-AHCP-OH

H-Phe-Orn-AHCP-OH

H-Phe-Phe-AHCP-OH

H-Phe-Pro-AHCP-OH

H-Phe-Ser-AHCP-OH

H-Phe-Thr-AHCP-OH

H-Phe-Tic-AHCP-OH

H-Phe-Trp-AHCP-OH

H-Phe-Tyr-AHCP-OH

H-Phe-Val-AHCP-OH.

Beispiel 7

Eine Lösung von 1 g BOC-Phe-His-AHCP-OMe [erhältlich durch Hydrogenolyse von BOV-Phe-(imi-BOM-His)-AHCP-OMe] in 20 ml Dioxan wird mit 20 ml 2 n Natronlauge versetzt. Man rührt 2 Std. bei 20°, stellt auf pH 3,5 ein und filtriert das erhaltene BOC-Phe-His-AHCP-OH ab; F. 206° (Zers.).

Analog erhält man durch Verseifung der entsprechenden Methylester die in Beispiel 2 angegebenen freien Carbonsäuren sowie:

BOC-Phe-Abu-AHCP-OH

BOC-Phe-Ada-AHCP-OH

BOC-Phe-Ala-AHCP-OH

BOC-Phe-Arg-AHCP-OH

BOC-Phe-Asn-AHCP-OH

BOC-Phe-Bia-AHCP-OH

BOC-Phe-Cal-AHCP-OH

BOC-Phe-Dab-AHCP-OH

BOC-Phe-Gln-AHCP-OH

BOC-Phe-Gly-AHCP-OH, Zers. bei 104-106°

BOC-Phe-N(im)-methyl-His-AHCP-OH

BOC-Phe-Ile-AHCP-OH

BOC-Phe-Leu-AHCP-OH

BOC-Phe-tert.-Leu-AHCP-OH

BOC-Phe-Lys-AHCP-OH

BOC-Phe-Met-AHCP-OH

BOC-Phe-αNal-AHCP-OH

BOC-Phe-βNal-AHCP-OH

BOC-Phe-Nbg-AHCP-OH

BOC-Phe-Nle-AHCP-OH, F. 196°

BOC-Phe-Orn-AHCP-OH

BOC-Phe-Phe-AHCP-OH

BOC-Phe-Pro-AHCP-OH

BOC-Phe-Ser-AHCP-OH

BOC-Phe-Thr-AHCP-OH

BOC-Phe-Tic-AHCP-OH

BOC-Phe-Trp-AHCP-OH

BOC-Phe-Tyr-AHCP-OH

BOC-Phe-Val-AHCP-OHz

Beispiel 8

Ein Gemisch von 1 g BOC-Phe-His-AHCP-OMe, 3 g $NH_4Cl$ und 100 ml gesättigter methanolischer $NH_3$-Lösung wird 4 Tage bei 20° stehengelassen. Man dampft ein, arbeitet wie üblich auf (pH 7) und erhält BOC-Phe-His-AHCP-$NH_2$, F. 198° - (Zers.).

Analog erhält man durch Reaktion der entsprechenden Ester mit $NH_3$ bzw. den entsprechenden Alkyl-oder Dialkylaminen die entsprechenden Amide, z. B.

BOC-Phe-His-AHCP-$NHCH_3$

BOC-Phe-His-AHCP-$NHC_2H_5$

BOC-Phe-His-AHCP-$NHC_4H_9$

BOC-Phe-His-AHCP-$NHCH_2CH_2CH(CH_3)_2$, F. 171-173°

BOC-Phe-His-AHCP-$N(CH_3)_2$

BOC-Phe-His-AHCP-$N(C_2H_5)_2$

BOC-Phe-His-AHCP-$N(C_4H_9)_2$

BOC-Phe-Abu-AHCP-$NH_2$

BOC-Phe-Dab-AHCP-$NH_2$

BOC-Phe-Lys-AHCP-$NH_2$

BOC-Phe-Met-AHCP-$NH_2$

BOC-Phe-N(im)-methyl-His-AHCP-$NH_2$

BOC-Phe-Nle-AHCP-$NH_2$

BOC-Phe-Orn-AHCP-$NH_2$

BOC-Ada-His-AHCP-$NH_2$

BOC-Cal-His-AHCP-$NH_2$

BOC-αNal-His-AHCP-$NH_2$

BOC-βNal-His-AHCP-$NH_2$

BOC-Trp-His-AHCP-$NH_2$

BOC-Tyr-His-AHCP-$NH_2$

BOC-Phe-Abu-AHCP-$N(CH_3)_2$

BOC-Phe-Dab-AHCP-$N(CH_3)_2$

BOC-Phe-Lys-AHCP-$N(CH_3)_2$

BOC-Phe-Met-AHCP-$N(CH_3)_2$

BOC-Phe-N(im)-methyl-His-AHCP-$N(CH_3)_2$

BOC-Phe-Nle-AHCP-N(CH₃)₂

BOC-Phe-Orn-AHCP-N(CH₃)₂

BOC-Ada-His-AHCP-N(CH₃)₂

BOC-Cal-His-AHCP-N(CH₃)₂

BOC-αNal-His-AHCP-N(CH₃)₂

BOC-βNal-His-AHCP-N(CH₃)₂

BOC-Trp-His-AHCP-N(CH₃)₂

BOC-Tyr-His-AHCP-N(CH₃)₂

## Beispiel 9

Man löst 1 g CBZ-Phe-His-AHCP-OH in 10 ml Methanol, hydriert an 0,5 g 10%ig. Pd-C bei 20° und 1 bar 3 Std. lang, filtriert, dampft ein und erhält H-Phe-His-AHCP-OH.

Analog sind die in Beispiel 6 angegebenen Verbindungen aus den entsprechenden CBZ-Derivaten erhältlich.

Die nachstehenden Beispiele betreffen pharmazeutische Zubereitungen.

## Beispiel A: Injektionsgläser

Eine Lösung von 100 g BOC-Phe-His-AHCP-OH und 5 g Dinatriumhydrogenphosphat in 3 l zweifach destilliertem Wasser wird mit 2n Salzsäure auf pH 6,5 eingestellt, steril filtriert, in Injektionsgläser abgefüllt, unter sterilen Bedingungen lyophilisiert und steril verschlossen. Jedes Injektionsglas enthält 500 mg Wirkstoff.

## Beispiel B: Suppositorien

Man schmilzt ein Gemisch von 500 g BOC-Phe-His-AHCP-OH mit 100 g Sojalecithin und 1400 g Kakaobutter, gießt in Formen und läßt erkalten. Jedes Suppositorium enthält 500 mg Wirkstoff.

## Ansprüche

1. Peptide der Formel I

X-Z-NR²-CHR³-CHOH-(CHR⁴)ₙ-CO-E I

worin

X H, R¹-O-$C_mH_{2m}$-CO-, R¹-$C_mH_{2m}$-O-CO-, R¹-$C_mH_{2m}$-CO-, R¹-SO₂-, (R¹-$C_mH_{2m}$)-L(R¹-$C_pH_{2p}$)-$C_rH_{2r}$-CO-, H-(NHCH₂CH₂)ₘ-NH-CH₂CO-oder 9-Fluorenyl-$C_m$ $H_{2m}$-O-CO-,

Z 1 bis 4 peptidartig miteinander verbundene Aminosäurereste ausgewählt aus der Gruppe bestehend aus Abu, Ada, Ala, Arg, Asn, Bia, Cal, Dab, Gln, Gly, His, N(im)-Alkyl-His, Ile, Leu, tert.-Leu, Lys, Met, αNal, βNal, Nbg, Nle, Orn, Phe, Pro, Ser, Thr, Tic, Trp, Tyr und Val,

E OH, OA, NH₂, NHA oder NA₂,

R¹ H, A, Ar, Ar-alkyl, unsubstituiertes oder ein-oder mehrfach durch Alkyl, Alkoxy und/oder Hal substituiertes Cycloalkyl mit 3-7 C-Atomen, Cycloalkylalkyl mit 4-11 C-Atomen, Bicycloalkyl oder Tricycloalkyl mit jeweils 7-14 C-Atomen, Bicycloalkylalkyl oder Tricycloalkylalkyl mit jeweils 8-18 C-Atomen, wobei die Gruppe (R¹-$C_mH_{2m}$)-L(R¹-$C_pH_{2p}$) auch Pyrrolidino, Piperidino, Morpholino oder Thiomorpholino bedeuten kann,

R² und R⁴ jeweils H oder A,

R³ Cycloalkylalkyl, Bicycloalkylalkyl oder Tricycloalkylalkyl mit jeweils bis zu 18 C-Atomen,

L CH oder N,

m, p und r jeweils 0, 1, 2, 3, 4 oder 5,

n 1 oder 2,

Ar unsubstituiertes oder ein-oder mehrfach durch A, AO, Hal, CF₃, OH und/oder NH₂ substituiertes Phenyl oder unsubstituiertes Naphthyl,

Hal F, Cl, Br oder J und

A Alkyl mit 1-8 C-Atomen bedeuten,

worin ferner an Stelle einer oder mehrerer -NH-CO-Gruppen auch eine oder mehrere -N(Alkyl)-CO-Gruppen stehen können,

sowie deren Salze.

2. a) 4-(BOC-Phe-His-amino)-5-cyclohexyl-3-hydroxypentansäure;

b) 4-(BOC-Phe-Nle-amino)-5-cyclohexyl-3-hydroxypentansäure;

c) 5-Cyclohexyl-3-hydroxy-4-(2-benzyl-4-phenylbutyryl-His-amino)-pentansäure.

3. Verfahren zur Herstellung eines Peptides der Formel I sowie von seinen Salzen, dadurch gekennzeichnet, daß man es aus einem seiner funktionellen Derivate durch Behandeln mit einem solvolysierenden oder hydrogenolysierenden Mittel in Freiheit setzt

oder daß man eine Verbindung, die der Formel I entspricht, aber an Stelle von H-Atomen eine oder mehrere zusätzliche C-C-und/oder C-N-und/oder C-O-Bindungen enthält, reduziert

oder daß man eine Carbonsäure der Formel II

X-G$^1$-OH II

worin G$^1$ (a) Z$^1$,

(b) Z bedeutet

mit einer Aminoverbindung der Formel III

H-G$^2$ III

worin

G$^2$ (a) Z$^2$-W-E-,

(b) W-E,

W -NR$^2$-CHR$^3$-CHOH-(CHR$^4$)$_n$-CO-und

Z$^1$ + Z$^2$ zusammen Z bedeuten

umsetzt,

und daß man gegebenenfalls in einer Verbindung der Formel I eine funktionell abgewandelte Amino- und/oder Hydroxygruppe durch Behandeln mit solvolysierenden oder hydrogenolysierenden Mitteln in Freiheit setzt und/oder einen Rest E durch Behandeln mit veresternden, solvolysierenden oder amidierenden Mitteln in einen anderen Rest E umwandelt und/oder eine Verbindung der Formel I durch Behandeln mit einer Säure oder Base in eines ihrer Salze überführt.

4. Verfahren zur Herstellung pharmazeutischer Zubereitungen, dadurch gekennzeichnet, daß man eine Verbindung der Formel I und/oder eines ihres physiologisch unbedenklichen Salze zusammen mit mindestens einem festen, flüssigen oder halbflüssigen Träger-oder Hilfsstoff und gegebenenfalls in Kombination mit einem oder mehreren weiteren Wirkstoff(en) in eine geeignete Dosierungsform bringt.

5. Pharmazeutische Zubereitung, gekennzeichnet durch einen Gehalt an mindestens einer Verbindung der Formel I und/oder einem ihrer physiologisch unbedenklichen Salze.

6. Verbindungen der Formel I und deren physiologisch unbedenkliche Salze zur Bekämpfung von Krankheiten.

7. Verwendung von Verbindungen der Formel I oder von deren physiologisch unbedenklichen Salzen bei der Bekämpfung der reninabhängigen Hypertension oder des Hyperaldosteronismus.

8. Verwendung von Verbindungen der Formel I oder von deren physiologisch unbedenklichen Salzen zur Herstellung eines Arzneimittels zur Bekämpfung der reninabhängigen Hypertension oder des Hyperaldosteronismus.